(19) Europäisches Patentamt
European Patent Office
Office européen des brevets

(11) **EP 1 985 326 B1**

(12) **EUROPÄISCHE PATENTSCHRIFT**

(45) Veröffentlichungstag und Bekanntmachung des Hinweises auf die Patenterteilung:
**15.06.2011 Patentblatt 2011/24**

(51) Int Cl.:
***A61M 16/00*** *(2006.01)*

(21) Anmeldenummer: **08400011.6**

(22) Anmeldetag: **14.03.2008**

(54) **Beatmungsgerät mit Ermittlung der alveolären Ventilation (a.V.)**

Respirator with determination of the alveolar ventilation (a.v.)

Appareil respiratoire avec une détermination de la ventilation alvéolaire

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **30.03.2007 DE 102007016031**

(43) Veröffentlichungstag der Anmeldung:
**29.10.2008 Patentblatt 2008/44**

(73) Patentinhaber: **Weinmann Geräte für Medizin GmbH & Co. KG**
**22525 Hamburg (DE)**

(72) Erfinder: **Göbel, Christoph**
**22457 Hamburg (DE)**

(74) Vertreter: **Klickow, Hans-Henning**
**Patentanwälte**
**Hansmann-Klickow-Hansmann**
**Jessenstrasse 4**
**22767 Hamburg (DE)**

(56) Entgegenhaltungen:
**WO-A-2005/051469 WO-A1-98/19818**

**Beschreibung**

Die Erfindung betrifft ein Beatmungsgerät mit einer Steuereinrichtung zur Durchführung eines Verfahrens zur Steuerung der Anzeige eines Beatmungsgerätes, bei dem mindestens ein Parameter der Beatmung messtechnisch erfasst und von einer steuereinrichtung ausgewertet wird, sowie bei dem mindestens ein Betriebsparameter des Beatmungsgerätes in Abhängigkeit vom messtechnisch erfassten Parameter verändert und als ein Parameter der Beatmung die alveoläre Ventilation des Benutzers ermittelt wird.

Die Erfindung betrifft darüber hinaus eine Vorrichtung zur Beatmung, die eine Steuereinrichtung, eine Atemgasquelle sowie mindestens einen Sensor zur Erfassung eines atmungsabhängigen Parameters aufweist.

Derartige verfahren und Vorrichtungen werden beispielsweise verwendet, um eine Optimierung der Betriebsweise von Beatmungsgeräten durchzuführen. Zielrichtung einer derartigen Optimierung kann beispielsweise eine Minimierung des Energieverbrauches, eine Minimierung von Lärmemissionen, eine Maximierung des Benutzungskomforts, eine Steigerung der Beatmungsqualität oder eine toleranzarme Einhaltung von vorgegebenen Beatmungsparametern sein. Anwendungen im medizintechnischen Bereich können beispielsweise bei BiLevel, PSV/PCV-, VCV-Beatmungen und anderen Formen und Arten der Beatmung, Heimbeatmung, Klinikbeatmung, CPAP- oder APAP-Beatmungen erfolgen. Ebenfalls sind im medizintechnischen Bereich Anwendungen bei mobilen oder stationären versorgungen von Patienten mit Sauerstoff oder mit Sauerstoff angereicherter Luft gegeben.

Es können aber auch diverse Anwendungen außerhalb des Gebietes der Medizintechnik erfolgen. Beispielsweise kann bei Beatmungsgeräten für Taucher oder Feuerwehrleute das Problem auftreten, daß bei einer Batteriespeisung der Energieverbrauch zu minimieren ist oder die akustische Wahrnehmung durch möglichst wenige Nebengeräusche beeinträchtigt werden soll. Das gleiche Problem kann auch im industriellen Bereich bei Tätigkeiten auftreten, die die Verwendung von Atmungsgeräten zum Schutz der betreffenden Person vor der Einwirkung von Umweltgasen erforderlich machen und bei denen die betreffenden Personen durch die vorgesehenen Tätigkeiten die Hände nicht frei haben, um selbst auf die verwendeten Beatmungsgeräte einwirken zu können. Schließlich sind auch Anwendungen im Bereich von Beatmungsgeräten denkbar, die von Astronauten oder Piloten verwendet werden. Bei Patienten mit respiratorischer Insuffizienz ist der Leitparameter der Einstellung der Kohlendioxid-Partialdruck, PaCO2. ziel der Beatmung ist es, den Patienten in den normokapnischen Bereich zu ventilieren, d.h. beispielsweise die Fähigkeit CO2 abzuatmen mit Hilfe des Beatmungsgerätes zu gewährleisten. Unter Normokapnie versteht man dabei allgemein die Erreichung eines arteriellen CO2-Partialdruckes um 40 mmHg (PaCO2 = 38 ... 42 mm Hg.

Nach dem Stand der Technik wird bei der Patienteneinstellung (Titration) auf eine Beatmung eine invasive Blutgasanalyse zur PaCO2-Ermittlung durchgeführt. Hierzu wird vor und nach der Beatmung eine Blutprobe entnommen und es werden die Blutwerte Sa02, Pa02, PaCO2, pH-Wert sowie die Konzentration des Basenüberschusses(BB) und Temperatur, Metabolite und Elektrolyte bestimmt.

Diese Methode hat mehrere Nachteile:

- Invasivität und damit Zufügen von Schmerzen für den Patienten,
- Momentbetrachtung der Blutgaswerte,
- Arbeitsaufwand personell und zeitlich,
- Beschränkung auf klinischen Einsatz,
- Kostenfaktor der Analyse (Zentrifuge Geräte, etc.).

Alternativ oder ergänzend kann die endexpiratorische CO2 - Konzentration in der Atemluft (petCO2) bestimmt werden.

Aus der WO 2005/051469 ist bereits ein Verfahren zur Brmittlung der alveolären Ventilation bekannt. Es wird hierzu ein Beatmungsgerät mit einer Steuereinheit beschrieben, das die alveoläre Ventilation des Benutzers meßtechnisch erfaßt. In Abhängigkeit von erfaßten Meßwerten wird mindestens ein Betriebsparameter des Beatmungsgerätes verändert. Aus dem Stand der Technik ist es ebenfalls bereits bekannt, die Atemfrequenz und das Tidalvolumen des Patienten zu berücksichtigen.

Aufgabe der vorliegenden Erfindung ist es, eine Vorrichtung der einleitend genannten Art derart zu verbessern, daß mindestens ein Zielparameter der Beatmung mit geringer Toleranz eingehalten wird.

Diese Aufgabe wird erfindungsgemäß durch das Beatmungsgerät nach Ansprunch 1 gelöst.

Ferner wird auch ein entsprechendes Verfahren beschrieben, wobei das Verfahren nicht zur Erfindung gehört. Über die Formel :

$$(1)\ \underline{Alveoläre\ Minutenventilation = (Tidalvolumen -}$$
$$\underline{Totraum)\ x\ Atemfrequenz}$$

lässt sich die alveoläre Ventilation (a.V.) für eine gegebene Situation ohne großen Aufwand abschätzen.

Erforderliche Daten wie die Atemfrequenz und das Tidalvolumen liegen in einem medizinischen Beatmungsgerät ohnehin vor.

Die Toträume (anatomischer Totraum und ggf. Toträume des verwendeten Patienteninterfaces), die zu einer Pendelluftbewegung führen, werden abgeschätzt.

Der anatomische Totraum kann zunächst spezifisch (d.h. bezogen auf 1 kg Körpergewicht) z.B. nach Numa über folgende Formel näherungsweise berechnet werden:

$$(2) \quad V_{anat\,Totraum} = 3.28 - 0.56x[\ln(1 + Alter)],$$

Das Alter wird dabei in Jahren angegeben.

[AH Numa et al.: Anatomic deadspace in infants and children. J Appl Physiol. 1996 May; 80(5): 1485-9] Für Erwachsene ergibt sich die einfache Annahme eines spezifischen Totrawavolumens von 2,2 ml/$kg_{Körpergewicht}$.

Für die Anwendung in einem Beatmungsgerät gibt der Arzt Alter und Körpergewicht des Patienten am Gerät ein, woraus dieses den theoretisch vorliegenden anatomischen Totraum in einer Näherung gemäß der Formel (2) berechnet.

Der (funktionelle) Maskentotraum kann darüber hinaus berücksichtigt werden, in dem der Anwender den Maskentyp am Gerät eingibt (wobei abgespeicherte Parameter herangezogen werden) oder indem der Anwender den Totraum aufgrund der Maskengröße und ggf. unter Zuhilfenahme einer Gebrauchsanweisung der Maske schätzt. Werden weitere Komponenten wie beispielsweise eine Gänsegurgel im Atemkreis verwendet, sind diese ebenfalls mit anzugeben oder abzuschätzen.

Die Totraumvolumina sind gemäß der Formel (1)- zur Ermittlung der alveolären Ventilation vom Tidalvolumen (VT) abzuziehen und mit der Atem- bzw. der Beatmungsfrequenz zu multiplizieren. Auf Basis aller Informationen können mittels eines Mikroprozessors Beispiel- Kombinationen für Tidalvolumina und Beatmungsfrequenzen errechnet werden, die zu einer hinreichenden alveolären Ventilation führen.

Um das Verfahren im Rahmen einer Beatmung für den Patienten angenehm zu gestalten, kann dieses beispielhaft in folgender Weise angewendet werden: Das Beatmungsgerät analysiert zu Beginn in einer kurzen CPAP- Phase (ähnlich wie im Weinmann TA- Modus), die Spontan- Atemfrequenz des Patienten. Hieraus können Startbedingungen für die Beatmung bezüglich der Parameter Frequenz sowie Inspirations- und Exspirationszeitanteile (I:E- Verhältnis) abgeleitet werden.

Das I:E- Verhältnis wird in einer Ausführungsform aus der Spontanatemkurve in die Beatmung übernommen.

Die Spontanatemfrequenz kann beim Übergang in die Beatmung zunächst beibehalten und in der Folge sukzessive angepasst werden. Ist die Beatmung wirksam, so steigt das Tidalvolumen durch die Beatmung an. Das Gerät kann dann, im Rahmen der zuvor als sinnvoll ermittelten Atemparameter, die Frequenz ggf. absenken, wenn dies angemessen erscheint.

Grundsätzlich wird eine Kombination aus Frequenz und Tidalvolumen mit einer ausreichenden (physiologischen) alveolären Ventilation angestrebt , bei der es nicht zu vorzeitigen frustranen Inspirationsbemühungen kommt. Durch die Überwachung von inspiratorischen Triggersignalen, also detektierten Einatembemühungen des Patienten, wird sichergestellt, dass es nicht zu einer Asynchronie unter der Beatmung kommen kann. Das Beatmungsgerät folgt hier vorzugsweise einer ggf. vom Patienten 'geforderten' höheren Frequenz. Es wird jedoch versucht, über höhere Beatmungsdrücke das Minutenvolumen sinnvoll zu steigern und die Atemfrequenz über eine verbesserte Ventilation im zweiten Schritt zu senken, sofern dies möglich ist. Typischerweise wird ein größeres Tidalvolumen bei tendenziell geringerer Frequenz zur Verminderung von Pendelluftbewegungen angestrebt.

Es ist auch vorstellbar, dass der Arzt einen konkreten Wert für die zu erzielende spezifische alveoläre Ventilation vorgibt, die von einem physiologischen Wert abweichen kann, in dem Sinne, in dem auch gelegentlich eine moderate Hyperkapnie klinisch zugelassen wird (die sogenannte ‚permissive Hyperkapnie') . Damit kann einer Beatmungssituation Rechnung getragen werden, in der sich physiologische Werte nicht erzielen lassen.

Das Beatmungsgerät kann zur Erzielung größerer Tidalvolumina, wie beschrieben, den Beatmungsdruck anheben. Dies geschieht vorzugsweise innerhalb von, seitens des Arztes, vordefinierten Grenzen. Hierbei wird der wirksame Beatmungsdruck dann niedrig gewählt, wenn sich so bereits eine gute alveoläre Ventilation erzielen lässt. Ist dies bei geringen Drücken nicht möglich, wird der wirksame Beatmungsdruck innerhalb der eingestellten Grenzen automatisch angehoben. Als wirksamer Beatmungsdruck ist hier vorzugsweise die Druckdifferenz zwischen Inspiration und Exspiration zu verstehen.

Ferner kann das Beatmungsverfahren verbessert werden, indem lungenmechanische Größen (Atemwegswiderstand/Resistance und Lungencompliance) mit berücksichtigt werden. Über eine Eingabe des Impedanztyps des Patienten (normal, restriktiv oder obstruktiv) kann auf einfache Weise Einfluss auf die Druckkurve genommen werden.

Alternativ können die Größen Resistance und Compliance auch gemessen und als diskrete Werte am Beatmungsgerät eingegeben werden.

Bei restriktiven Erkrankungen wird der Druck erst zum Ende der Inspiration hin auf das Maximum gefahren, wodurch sich ein flacherer, für den Patienten angenehmerer Druckverlauf ergibt, wohingegen bei obstruktiven Erkrankungen ein steilerer Druckanstieg gewählt wird.

Mittels der vorgestellten Methode können geeignete Einstellungen für eine kontrollierte Beatmung (PCV, T-Modus, VCV) ermittelt werden oder alternativ eine Hintergrundfrequenz und I:E- Verhältnis, zusammen mit einem geeigneten Druckniveau) für einen ST Modus bzw. PSV mit dem Ziel, physiologische Werte für die alveoläre Ventilation zu erzielen. Das Verfahren lässt sich also mit verschiedenen bekannten Beatmungsverfahren kombinieren.

Es ist ebenfalls möglich und sinnvoll, einen Alarm bei mangelnder alveolärer Ventilation auszulösen, wie sie zum Beispiel bei Hechelatmung ("rapid shallow breathing") auftritt. Hierbei kann z.B. ein spezifischer Wert in ml/min/Kg-Körpergewicht (zusammen mit dem Körpergewicht) als Alarmgrenzwert eingegeben werden. Dies bedeutet für den Arzt eine an Standardwertn orientierte und damit erheblich vereinfachte Einstellung gegenüber einer patientenindividuellen Auswahl von Alarmgrenzwerten.

Grundsätzlich ist bei Anwendung des Verfahrens zu beachten, dass eine theoretisch ermittelte alveoläre Ventilation nur bei gesundem Lungengewebe, und ausreichender Lungenperfusion auf zu erwartende Blutgaswerte schließen lässt. Bei diffusiven Störungen des Lungenparenchyms oder bei einem vorliegenden , Ventilations/Perfusions-Missverhältnis' ist diese Übertragung auf einfache Weise nicht möglich.

Zur weiteren Optimierung des Verfahrens ist daher eine Prüfung und/oder Optimierung der Beatmung angedacht: Hierzu werden im Rahmen einer invasiven Blutgasanalyse gewonnene Werte am Beatmungsgerät eingegeben (beispielsweise ohne und unter der Beatmung), wodurch ein Abgleich mit den theoretisch ermittelten Werten der alveolären Ventilation ermöglicht wird. Im Beatmungsalgorithmus kann damit eine patientenspezifische Anpassung erfolgen, z.B. indem bei Erkrankungen des Lungenparenchyms ein erhöhter Zielwert für die alveoläre Ventilation angestrebt wird.

In einem weiteren Beispiel des Verfahrens ist folgendes möglich:

Die Errechnung des Totraumvolumens kann auf Basis der sogenannten Bohrformel ergänzend oder zur weiteren Optimierung des Verfahrens abgeglichen oder ersetzt werden durch eine Berechnung, die auf einer Blutgasanalyse ($pa_{CO2}$) und einer exspiratorischen (endtidalen) CO2- Messung ($Pet_{CO2}$) sowie der Ermittlung des exhalierten Volumens $V_E$ basiert:

$$(3) \quad V_{physiol.Totraum} = V_E * \left( Pa_{CO2} - Pet_{CO2}^{\cdot} \right) \cdot / \cdot Pa_{CO2}$$

Auf diese Weise lässt sich der abgeschätzte Totraum durch einen aus Messgrößen ermittelten Wert für den physiologischen Totraum ersetzen, wobei ggf. zusätzlich vorliegende alveoläre Totraumvolumina (resultierend aus einem Ventilations- Perfusions- Missverhältnis) mit berücksichtigt werden!

Bei Einsatz einer exspiratorischen CO2- Messung ist ebenfalls vorstellbar, dass diese am Exhalationsort (Ausatemsystem, Ausatemventil) zum Einsatz kommt. Der Umstand, dass die Gaszusammensetzung im Totraum sich von der in den Alveolen unterscheidet, wird dabei genutzt. Die Signale Flow (Q[t]) und Konzentrationsveränderung des CO2 (fexsp.CO2 = f[t]) als Funktion der Zeit liefern Informationen über den gesamten wirksamen Totraum (jedoch ohne evtl. vorhandene Alveolar-Toträume), indem das Integral des exspiratorischen Flows vom Beginn bis zu einem messbaren Anstieg des CO2 [ $t(f_{exsp.CO2} > 0)$ ] gebildet wird.

$$(4) \quad V_{Totraum} \text{ (anatomisch + Patienteninterface) :}$$

$$V_{Totraum} = \int_{t_{exsp}=0}^{t(f_{exsp.CO2}>0)} Q \cdot dt$$

Die Messung der endexspiratorischen $CO_2$- Fraktion kann ebenfalls direkt als Regelparameter dienen, da sich bei vermehrter Totraumventilation (also ungünstigem Verhältnis $V_{Totraum}/V_{Tidalvolumen}$ )eine höhere endtidale $CO_2$ - Konzentration ergibt. Ggf. kann also über eine Anpassung des wirksamen Beatmungsdruckes auch auf den endexspiratorischen $CO_2$ - Partialdruck ($P_{etCO2}$) in der Atemluft geregelt werden, um die alveoläre Ventilation zu optimieren.

Für den Fall, dass unter der Beatmung zusätzlich Sauerstoff gegeben wird, ist die Berechnung der alveolären Ventilation ggf. zu korrigieren, wobei sich diese Korrektur naturgemäß nur auf den 02- Austausch und nicht auf die Abatmung von $CO_2$ beziehen kann.

Typischerweise werden in Beatmungsgeräten patientenbezogene Daten gespeichert. Mit dem vorgestellten Verfahren steht ein weiterer Parameter zur Verfügung, der zusätzlich als Verlaufswert unter der Beatmung oder als gemittelter Wert abgespeichert und zu einem späteren Zeitpunkt wieder angezeigt werden kann. Dieser Wert kann bei Bedarf - z.B. zur Bewertung einer nächtlichen Beatmung - am Gerät abgerufen werden. Auf diese Weise ist es möglich, Schwankungen der alveolären Ventilation während einer Beatmungsphase wiederzugeben. Die a.V. kann z.B. als Kurve dargestellt werden, womit sich sehr einfach Phasen einer Hypoventilation erkennen lassen. Damit lässt sich auch eine Korrelation zwischen einer Blutgasanalyse, die zu einem diskreten Zeitpunkt durchgeführt wird, und dem Verlauf der a.V. herstellen. Dies erlaubt eine Bewertung, ob eine durchgeführte Blutgasanalyse einen zufälligen Wert zu einem bestimmten Zeitpunkt ergibt oder einen repräsentativen Wert für einen längeren Beatmungszeitraum widerspiegelt.

Es ist auch daran gedacht, das Verfahren im Sinne der Überwachung eines herkömmlichen Beatmungsmodus einzusetzen. Dabei können dem Arzt Hinweise auf vorhandene Optimierungsmöglichkeiten der Beatmung gegeben werden im Sinne einer Anpassung von Parametern oder wenn das Patienteninterface gegen ein günstigeres (im Sinne verminderter Toträume) ausgetauscht werden sollte.

Wird das Verfahren zur Titration eingesetzt, ist daran gedacht, dass dieses im Sinne einer automatischen Einstellung von Beatmungsparametern für den Patienten, längere Zeit andauern kann. In dieser zeit wird eine Reihe von Parametern systematisch oder zufällig verändert. Dabei zeichnet das Gerät die spezifischen Reaktionen des Patienten, auf die veränderten Parameter, in einem Speicher auf. Aus dieser patienten-individuellen Lernphase, gewinnt das Gerät somit Informationen, die später für die Beatmung des Patienten herangezogen werden können. Insbesondere ist erfindungsgemäß daran gedacht, dass das Gerät für den Patienten optimierte Beatmungseinstellungen selbst vornimmt und/oder dem Arzt Hinweise für optimierte Beatmungseinstellungen anbietet. Dieser Vorgang bedeutet für den behandelnden Arzt in der Praxis eine erhebliche Zeitersparnis und ist auch umfassender, da der Arzt selbst in der klinischen Praxis nur eine sehr begrenzte Zeit für die Einstellung eines Beatmungsgerätes zur Verfügung hat.

Es ist auch daran gedacht, dass als Ergebnis der Berechnung und nachfolgender Beatmungs- ‚Versuche' dem Arzt der Vorschlag 'gemacht' wird, eine kleinere Maske zu verwenden, um ggf. das Totraumvolumen zu optimieren, wenn andere Parameter- 'Permutationen' nicht zielführend waren oder wenn bereits zu Beginn erkannt wird, dass ein Maskentyp über ein ungünstig großes Totraumvolumen für einen bestimmten Patienten verfügt.

Es ist ferner daran gedacht, dass Informationen aus ermittelten Atemkurven für die weitere Anpassung und Optimierung der Beatmung herangezogen werden. Beispielsweise kann bei einer kontrollierten Beatmung an exspiratorischen Flowverläufen erkannt werden, ob es zu vorzeitigen Atemhüben durch das Beatmungsgerät kommt, wodurch die Gefahr einer dynamischen Hyperinflation provoziert würde und woraufhin die Exspirationszeit bzw. das I:E- Verhältnis angepasst werden würde.

Das Verfahren kann insbesondere im Bereich der pädiatrischen Beatmung von großem Nutzen für den Anwender sein, da es hier - abhängig vom Tidalvolumen eines Kindes und der verwendeten Maske - leicht zu einem hohen Anteil an reiner Pendelluft kommen kann, die dem Anwender nicht unbedingt anschaulich einsichtig ist. Hier ist die Auswahl eines geeigneten Interfaces von besonderer Bedeutung und eine entsprechende Hilfestellung bei der Auswahl durch den Algorithmus möglich.

Es ist vorgesehen, dass der Lernmodus vorübergehend zur Titration zum Einsatz kommt und eine Beamtungseinstellung dann 'eingefroren' wird. Alternativ ist auch vorstellbar, dass ein Patient dauerhaft im 'Lern'- Modus über längere Zeit beatmet wird, um mehr Flexibilität zu erzielen.

Ergänzend oder alternativ kann das Beatmungsgerät durch (z.B. transkutan) gemessene Blutgaswerte weitere Steuerparameter erhalten. Dabei ist jedoch zu beachten, dass auf der Haut angebrachte Sensoren (z.B. Ohrclip) während der Anwendung verrutschen und Fehlsignale provozieren können. Im Vergleich dazu bietet das vorgestellte Verfahren ggf. eine erhöhte Sicherheit oder kann bei unplausiblen Sensorsignalen als Backup- Steuerung dienen.

Wenn das Totraumvolumen eines Patienten bekannt ist, kann dieses am Beatmungsgerät ebenso eingegeben und für die Ermittlung sinnvoller Beatmungsparameter herangezogen werden.

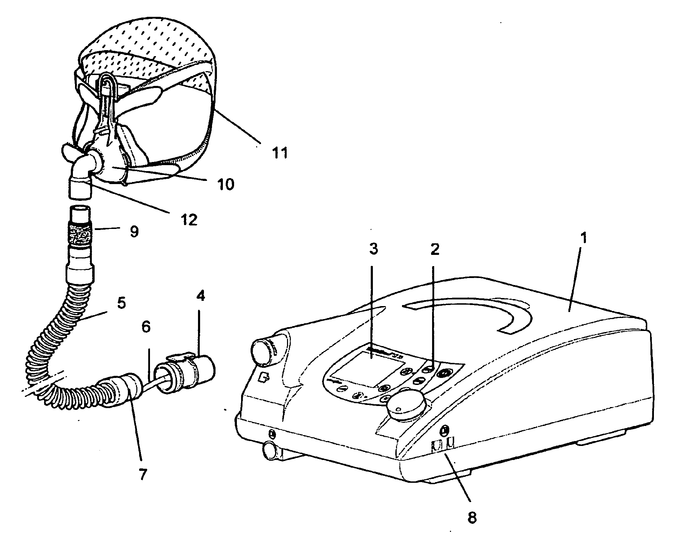

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Atemgaspumpe angeordnet. Über eine Kopplung (4) wird ein Verbindungsschlauch (5) angeschlossen. Entlang des Verbindungsschlauches (5) kann ein zusätzlicher Druckmeßschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf. Ein Anfeuchter kann adaptiert werden.

Im Bereich einer dem Gerätegehäuse (1) abgewandten Ausdehnung des Verbindungsschlauches (5) ist ein Ausatmungselement (9) angeordnet. Ebenfalls kann ein Ausatemventil verwendet werden.

Fig. 1 zeigt darüber hinaus ein als Beatmungsmaske (10) ausgebildetes Patienten-Interface, das als Nasalmaske realisiert ist. Eine Fixierung im Bereich eines Kopfes eines Patienten kann über eine Kopfhaube (11) erfolgen. Im Bereich ihrer dem Verbindungsschlauch (5) zugewandten Ausdehnung weist das Patienten-Interface (10) ein Kupplungselement (12) auf.

Die Eingabe und / oder Ausgabe von Daten wie beispielsweise Alter und Körpergewicht kann über das Bedienfeld (2) und die Anzeige (3) direkt am Beatmungsgerät oder über die Schnittstellen (8) erfolgen. Die Schnittstellen können kabelgebunden, als Infrarot-Schnittstelle, als Bluetooth-Schnittstelle oder USB realisiert sein. Im Bereich eines Gerätegehäuses kann ein Sauerstoffzuschaltventil an die Vorrichtung zur Beatmung adaptiert werden. Es ist denkbar das Atemgas zusätzlich mit Sauerstoff anzureichern, um die Patientenversorgung zu verbessern.

Zudem können in einem der Geräte Schnittstellen zu Drittgeräten und Informations-Management-Systemen beispielsweise zur Aufnahme von Speichermedien, zur Verbindung mit einem EKG, EEG, Drucker, Defibrillator etc. vorgesehen werden.

Über ein Modem oder eine andere Schnittstelle können ebenfalls aufgezeichnete Daten, wie Trends, außerordentliche Ereignisse, Warnmeldungen etc. dem Arzt, sowie Auffälligkeiten, Betriebsstunden oder andere zur Gewährleistung der einwandfreien Funktion dem Wartungs-/Kundendienst bei Bedarf übermittelt werden.

**Patentansprüche**

1. Beatmungsgerät mit einer Steuereinheit, die gemäß einem Verfahren betrieben wirt, bei dem mindestens ein Parameter der Beatmung messtechnisch erfasst und von einer Steuereinrichtung ausgewertet wird, sowie bei dem mindestens ein Betriebsparameter des Beatmungsgerätes in Abhängigkeit vom messtechnisch erfassten Parameter verändert und als ein parameter der Beatmung die alveoläre Ventilation den Benutzere ermittelt wird und zur Berechnung des anatomischen Totraumvolumens eines Patienten eine Alterseingabe erfolgt und die Berechnung entsprechend einer hinterlegten Formel unter Berücksichtigung des Alters und des Körpergewichts des Patienten erfolgt und dazu das Alter und Körpergewicht des Patienten über das Bedienfeld (2) und die Anzeige (3) direkt am Beatmungegerät oder ülber die Schnittstellen (8) eingegeben wird
**dadurch gekennzeichnet, dass**
diskrete werte und/oder verlanfswerte der alveolären ventilation zur Dokumentation einer Beatmungssituation angereigt werden, wobei die verlaufswerte unter der Beatmung oder als gemittelte werte abgespeichert und zu einem späte- ren Zeitpunkt angezeigt worden, wobei der werft am Gerät abrufbar ist und wobei ein Alarm bei einer mangelnden alveolären Ventilation ausgelöst wird
wobei dazu ein spezifischer Wert in ml/min/Kg Körpergewicht, zuaammen mit dem Körpergewicht, als Alermgrenzwert eingegeben wird.

**Claims**

1. Respirator comprising a control unit which is operated according to a method in which at least one respiration parameter is detected metrologically and is evaluated by a control device, and in which at least one operating parameter of the respirator is modified according to the parameter which has been detected metrologically, and the alveolar ventilation of the user is determined as a respiration parameter and, for calculating the anatomic dead space volume of a patient, an age is entered and the calculation is made according to a formula which has been provided, taking into account the age and the body weight of the patient, and to this end the age and body weight of the patient is entered via the control panel (2) and the display (3) directly into the respirator or via the interfaces (8),
**characterised in that**
discrete values and/or historical values of the alveolar ventilation are displayed in order to document a respiration situation, the historical values being stored during respiration or as averaged values and being displayed at a later time, the value being able to be recalled on the device and an alarm being triggered when alveolar ventilation is insufficient, a specific value in ml/min/Kg body weight, together with the body weight, being entered as an alarm limit value.

**Revendications**

1. Appareil respiratoire, avec une unité de commande, qui est entraînée par un procédé, dans lequel un paramètre de la respiration est saisi selon une technique de mesure et est évalué par un dispositif de commande, ainsi que dans lequel au moins un paramètre de fonctionnement dudit appareil respiratoire est modifié en fonction du paramètre saisi selon la technique de mesure, et la ventilation alvéolaire de l'utilisateur est déterminée en tant que paramètre de la respiration, et dans lequel, pour le calcul du volume de l'espace mort anatomique d'un patient, une introduction de l'âge est effectuée et le calcul est exécuté selon une formule enregistrée, compte tenu de l'âge et du poids dudit patient, et l'âge et le poids du patient étant introduits en plus, au moyen du tableau de commande (2) et de l'affichage (3), directement sur l'appareil respiratoire ou par l'intermédiaire de l'interface (8), **caractérisé en ce que** des valeurs discrètes et / ou des valeurs de l'évolution de la ventilation alvéolaire sont affichées pour la documentation d'une situation de ventilation, les valeurs de l'évolution étant mémorisées sous la ventilation ou en tant que valeurs moyennes et affichées à un moment ultérieur, la valeur pouvant être extraite sur l'appareil et une alarme étant déclenchée en cas de ventilation alvéolaire insuffisante, sachant que, de plus, une valeur spécifique est introduite en ml / min / kg, en commun avec le poids, en tant que valeur limite d'alarme.

11

10

12

9

3    2    1

5

4

6

8

7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 051469 A

**In der Beschreibung aufgeführte Nicht-Patentliteratur**

- **AH Numa et al.** Anatomic deadspace in infants and children. *J Appl Physiol.,* Mai 1996, vol. 80 (5), 1485-9